# FASCICULE DE BREVET EUROPEEN

(11) **EP 3 661 409 B1**
(45) Date de publication et mention de la délivrance du brevet: **27.03.2024**
(21) Numéro de dépôt: 18762573.6
(22) Date de dépôt: 19.07.2018
(51) Int. Cl.: A61B 5/00, A61B 5/145, A61B 5/1455, A61B 17/24, A61M 29/02, A61B 17/12

(54) **BALLONNET GONFLABLE A USAGE MÉDICAL**
AUFBLASBARER BALLON FÜR MEDIZINISCHE ZWECKE
INFLATABLE BALLOON FOR MEDICAL USE

(30) Priorité: 03.08.2017 FR 1757465
(43) Date de publication de la demande: 10.06.2020
(73) Titulaire: DIANOSIC, 67000 Strasbourg (FR)
(72) Inventeur: AUGUSTIN, Marc, 75015 Paris (FR); BASTIDE, Philippe, 92130 Issy-les-Moulineaux (FR)
(74) Mandataire: Bonnet, Michel
(86) Numéro de dépôt international: PCT/FR2018/051847
(87) Numéro de publication internationale: WO 2019/025694

(56) Documents cités:
- WO-A1-2016/179563
- WO-A1-2017/064437
- US-A1- 2007 161 851
- US-A1- 2010 087 782
- US-A1- 2011 010 925
- US-A1- 2012 245 553

## Description

La présente invention concerne un ballonnet gonflable à usage médical, destiné à être inséré dégonflé dans une cavité de corps humain ou animal puis gonflé à l'intérieur de cette cavité en vue de faire pression contre les parois de cette cavité.

En otorhinolaryngologie par exemple, un ballonnet de ce type est inséré dans la cavité nasale d'un patient, puis gonflé une fois correctement positionné à l'aide d'un fluide tel que de l'air, de l'eau gélifiée ou du sérum physiologique. Une application courante de ce type de ballonnet est le traitement d'hémorragies par pression contre les parois internes de la cavité nasale.

L'invention s'applique plus particulièrement à un ballonnet à usage médical, destiné à être inséré dégonflé dans une cavité de corps humain ou animal puis gonflé à l'intérieur de cette cavité en vue de faire pression contre elle, comportant :
- un corps de ballonnet dont la paroi est en matériau souple gonflable,
- une ouverture de gonflage pour l'introduction d'un fluide dans le corps de ballonnet de manière à le gonfler sous pression, et
- au moins un dispositif de mesure d'une grandeur biologique de tissus biologiques de la cavité et/ou de fluides biologiques présents dans cette cavité.

Ce type de ballonnet est par exemple décrit dans la demande de brevet internationale PCT publiée sous le numéro WO 2016/067153 A1. Dans cette publication, le dispositif de mesure est destiné à mesurer la saturation « pulsée » en oxygène, également appelée SpO2.

De manière générale, il peut être délicat de placer un dispositif de mesure sur la face externe d'un corps de ballonnet. En effet, le dispositif de mesure doit dépasser le moins possible afin de ne pas gêner l'écoulement des fluides dans la cavité et/ou de ne pas irriter les tissus biologiques de la cavité. Or, il est nécessaire de prévoir un boîtier pour protéger l'électronique du dispositif de mesure des agressions du milieu biologique de la cavité, comme cela est décrit par exemple dans la demande de brevet chinois publiée sous le numéro CN 104055525 A. La présence de ce boîtier vient augmenter la taille du dispositif de mesure et augmente son encombrement, de sorte qu'il est difficile de réaliser un dispositif de mesure de faible saillance. Le document US 2012/0245553 A1 divulgue un ballonnet gonflable avec un contrôleur électronique couplé à la paroi du ballonnet et attenant à une valve configurée pour réguler l'administration au sujet du fluide contenu dans le ballonnet.

Il peut ainsi être souhaité de prévoir un ballonnet gonflable à usage médical qui permette de s'affranchir d'au moins une partie des problèmes et contraintes précités.

Il est donc proposé un ballonnet à usage médical, destiné à être inséré dégonflé dans une cavité de corps humain ou animal puis gonflé à l'intérieur de cette cavité en vue de faire pression contre elle, comportant :
- un corps de ballonnet dont la paroi est en matériau souple gonflable,
- une ouverture de gonflage formée pour l'introduction d'un fluide dans le corps de ballonnet de manière à le gonfler sous pression, et
- au moins un dispositif de mesure d'une grandeur biologique de tissus biologiques de la cavité et/ou de fluides biologiques présents dans cette cavité,
caractérisé en ce que le dispositif de mesure comporte :
- un capteur externe fixé sur une face externe de la paroi du corps de ballonnet et conçu pour fournir un signal électrique de mesure sensible à la grandeur biologique mesurée, et
- un module interne situé à l'intérieur du corps de ballonnet et comportant un dispositif de traitement conçu pour traiter le signal électrique de mesure afin de fournir au moins une mesure de la grandeur biologique.

Ainsi, seul le capteur externe dépasse de la face externe du corps de ballonnet, de sorte que le dispositif de mesure présente une saillance réduite. En outre, il n'est plus nécessaire de prévoir un boîtier de protection du module interne, puisque ce dernier est dans un environnement non agressif, généralement de l'air, de l'eau gélifiée ou du sérum physiologique.

De façon optionnelle, le capteur externe transmet le signal électrique de mesure au module interne au moyen d'un ou plusieurs fils électriques passant dans un ou des conduits ménagés au travers de la paroi du corps de ballonnet.

De façon optionnelle également, un matériau d'étanchéité obstrue le ou les conduits.

De façon optionnelle également, le matériau d'étanchéité comporte de la colle fixant le capteur à la face externe de la paroi du corps de ballonnet.

De façon optionnelle également, le module interne est fixé sur une face interne de la paroi du corps de ballonnet.

De façon optionnelle également, le matériau d'étanchéité comporte de la colle fixant le module interne à la face interne de la paroi du corps de ballonnet.

De façon optionnelle également, le module interne comporte en outre un dispositif de communication sans fil conçu pour communiquer la mesure de la grandeur biologique à un dispositif situé à l'extérieur du corps humain ou animal dans lequel le ballonnet gonflable est inséré.

De façon optionnelle également, le module interne comporte en outre une mémoire pour stocker la mesure de la grandeur biologique.

De façon optionnelle également, le capteur externe est imprimé sur la face externe de la paroi du corps de ballonnet.

De façon optionnelle également, le ballonnet gonflable à usage médical est à utilisation en otorhinolaryngologie ou en neurochirurgie et le corps de ballonnet est préformé pour épouser les formes intérieures d'une cavité nasale de corps humain ou animal lorsqu'il est gonflé.

L'invention sera mieux comprise à l'aide de la description qui va suivre, donnée uniquement à titre d'exemple et faite en se référant aux dessins annexés dans lesquels :
- la figure 1 représente schématiquement la structure générale d'un ballonnet gonflable à usage médical, selon un mode de réalisation de l'invention,
- la figure 2 illustre un exemple de forme pour le ballonnet de la figure 1,
- les figures 3 et 4 illustrent des dispositions du ballonnet de la figure 1 dans une cavité nasale, avant et après gonflage,
- les figures 5 et 6 représentent schématiquement la structure générale d'un ballonnet gonflable à usage médical, selon un autre mode de réalisation de l'invention, avant et après gonflage, et
- la figure 7 représente schématiquement un dispositif de mesure d'une grandeur biologique, fixé à une paroi du ballonnet gonflable.

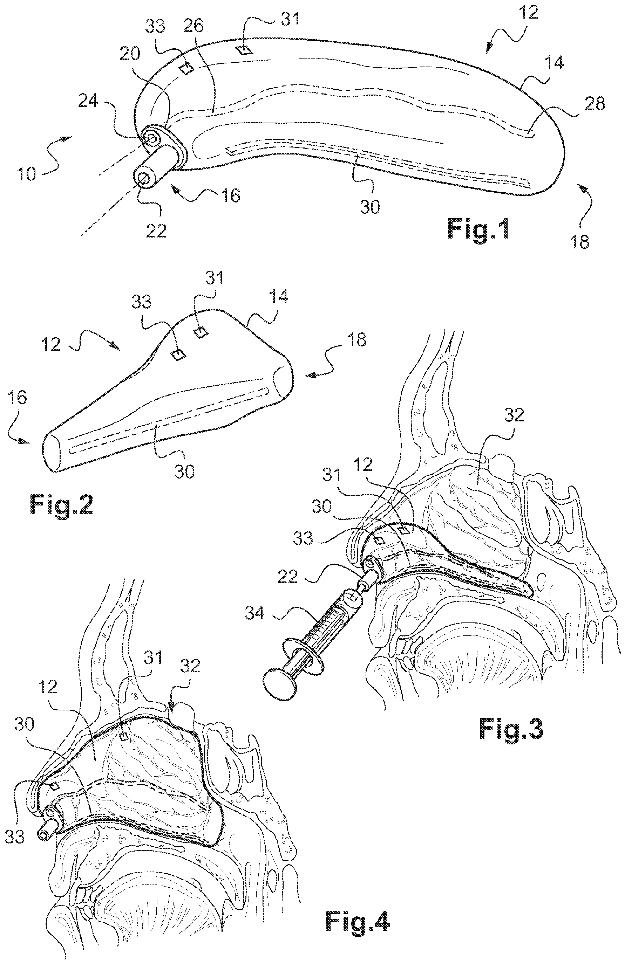

Le ballonnet gonflable 10 à usage médical représenté schématiquement sur la figure 1 comporte un corps de ballonnet 12 dont la paroi 14 est en matériau souple gonflable, par exemple en silicone ou tout matériau équivalent présentant ces propriétés. Il est destiné à être inséré dégonflé dans une cavité de corps humain ou animal, par exemple la fosse nasale, puis gonflé à l'intérieur de cette cavité en vue de faire pression contre elle. Il est de forme générale allongée, présentant une extrémité proximale 16 de gonflage et une extrémité distale 18 de positionnement en fond de cavité. L'extérieur de la paroi 14 est avantageusement enduit d'un produit glissant et non agressif pour les tissus biologiques de la cavité.

L'extrémité proximale 16 comporte un support rigide 20 qui présente une première ouverture et un embout de gonflage 22 menant à cette première ouverture. Cet embout 22 facilite l'introduction d'une seringue pour gonfler le corps de ballonnet 12 sous pression à l'aide d'un fluide tel que de l'air, de l'eau gélifiée, du sérum physiologique, ou tout autre fluide approprié. Il peut être muni d'une valve, ou de tout système approprié pour le gonflage, de façon à permettre une injection ou une aspiration de fluide sans qu'il n'y ait de fuites lorsque l'on enlève la seringue ou le dispositif de gonflage.

Le support rigide 20 présente en outre de façon optionnelle une seconde ouverture 24 destinée à l'introduction d'un endoscope dans le corps de ballonnet 12. Cette seconde ouverture 24 se présente par exemple sous la forme d'une valve ou de tout système permettant l'introduction d'un endoscope tout en empêchant toute sortie de fluide introduit dans le corps de ballonnet 12.

Dans ce cas, il est avantageux, voire nécessaire que le fluide soit le plus transparent ou translucide possible pour une meilleure observation de l'environnement par l'endoscope à l'intérieur du ballonnet 10.

Dans ce cas également, le ballonnet 10 peut en outre comporter une chaussette allongée 26 s'étendant à l'intérieur du corps de ballonnet 12 et présentant une extrémité distale fermée 28 non solidaire de la paroi 14 du corps de ballonnet 12. Une chaussette allongée doit être comprise comme un capuchon allongé, de forme complémentaire à celle de l'endoscope, à l'intérieur duquel débouche la seconde ouverture 24. Elle permet l'introduction de l'endoscope sans contact de ce dernier avec le fluide présent dans le corps de ballonnet 12. Elle est avantageusement formée dans un matériau souple éventuellement élastique, par exemple en silicone ou tout matériau de même nature.

La paroi 14 du corps de ballonnet 12 présente localement une portion allongée 30, dite semelle, de plus forte rigidité que le reste de la paroi 14, cette semelle 30 s'étendant de l'extrémité proximale 16 jusqu'à l'extrémité distale 18. Elle forme la base du corps de ballonnet 12, facilitant l'introduction et le maintien du ballonnet 10 dans la cavité souhaitée même lorsque celui-ci est dégonflé. Par ailleurs, elle remplit une fonction de guide lors du gonflage du ballonnet 10, en lui donnant une direction précontrainte.

En pratique la semelle 30 peut être une pièce rapportée contre la paroi 14 du corps de ballonnet 12, constituée de tout matériau assurant la fonction de rigidité voulue. Ce matériau peut être choisi de façon non limitative parmi des polymères tels que du polychlorure de vinyle, du polysiloxane, du polyuréthane, du polycarbonate polyéthylène, du polyméthacrylate de méthyle, du polytéréphtalate d'éthylène, ou parmi des polymères fluorés tels que du polytétrafluoroéthylène ou du polychlorotrifluoroéthylène. De façon alternative, elle peut être venue de matière avec le reste de la paroi 14 du corps de ballonnet 12, mais elle présente alors une épaisseur sensiblement supérieure au reste de cette paroi 14 pour augmenter sa rigidité.

Le ballonnet gonflable 10 comporte par ailleurs un ou plusieurs dispositifs de mesure 31 in situ d'au moins une grandeur biologique des tissus biologiques de la cavité nasale et/ou des fluides biologiques présents dans cette dernière. La grandeur biologique comporte par exemple un taux d'hémoglobine, un taux de protéines, un taux de glucose, un taux d'acide lactique ou un taux de lactate.

Le ballonnet 10 peut en outre comporter un ou plusieurs dispositifs de mesure 33 de la pression interne du ballonnet 10.

Comme illustré sur la figure 2, le ballonnet 10 peut être conçu pour un usage en otorhinolaryngologie ou en neurochirurgie. Dans ce cas, la paroi 14 du corps de ballonnet 12 peut être préformée pour épouser les formes intérieures d'une cavité nasale de corps humain ou animal en conformation gonflée.

La figure 3 illustre le positionnement du ballonnet 10 des figures 1 et 2 dans une cavité nasale 32, en conformation dégonflée. Grâce à la semelle 30 de rigidité supérieure au reste de la paroi 14 du corps de ballonnet 12, le ballonnet 10 est facilement introduit dans la cavité nasale 32. Une fois positionné, un fluide est introduit par l'embout 22 dans le corps de ballonnet 12 à l'aide d'une seringue 34. En procédant de la sorte, la pression à l'intérieur du corps de ballonnet est facilement contrôlée.

Cela permet le gonflage du ballonnet 10 jusqu'à obtention du résultat illustré sur la figure 4 selon lequel le corps de ballonnet 12 occupe tout l'espace intérieur de la cavité nasale 32 en épousant la forme de ses parois.

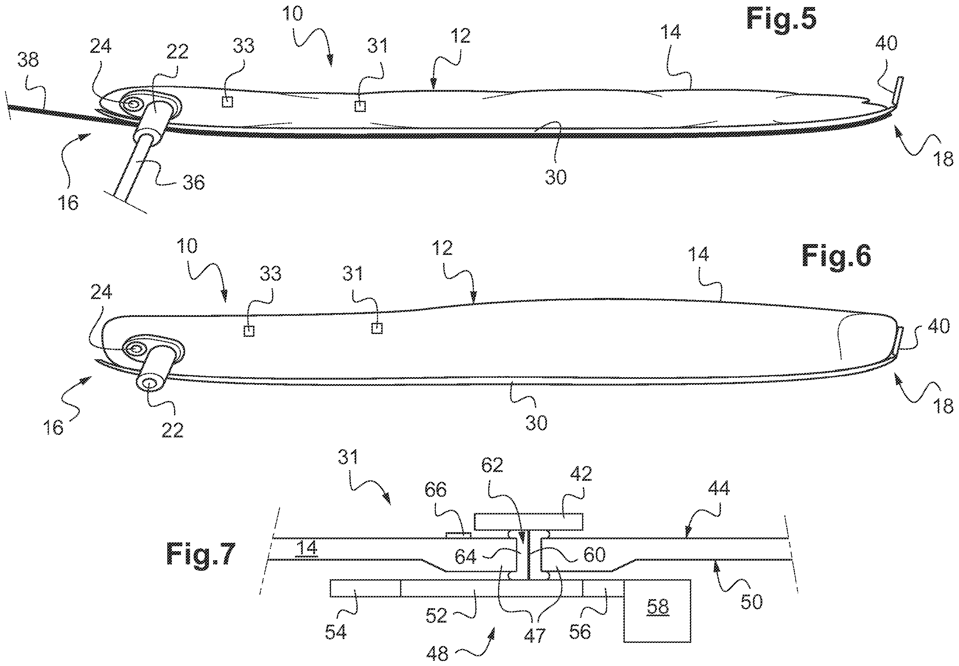

Selon un autre mode de réalisation illustré en coupe sur la figure 5, et en reprenant les mêmes références que précédemment pour les éléments inchangés, le ballonnet 10 comporte en outre une tige rigide 36 d'introduction amovible s'étendant vers l'extérieur dans le prolongement de l'embout 22. Cette tige rigide 36 permet de faciliter l'installation du ballonnet 10 dans la cavité et peut être rompue ou retirée lorsque cette installation est correctement réalisée. Le ballonnet 10 peut être gonflé à l'aide de la seringue 34 avant ou après cette manoeuvre.

Le ballonnet 10 de cet autre mode de réalisation comporte en outre un fil rigide amovible 38 de mise en place, s'étendant en renforcement de la semelle 30 le long de ou dans cette dernière. Il est par exemple conçu en alliage de nickel-titane, ce matériau possédant des propriétés intéressantes de mémoire de forme et d'élasticité.

Enfin, le ballonnet 10 de cet autre mode de réalisation comporte un renfort 40 en extrémité distale 18 dans le prolongement de la semelle 30. Ce renfort distal 40 présente, comme la semelle 30, une rigidité supérieure au reste de la paroi 14 du corps de ballonnet 12 et est conformé, par exemple selon un angle proche de 90° par rapport à la semelle 30, de manière à empêcher, en coopération avec cette dernière, tout allongement du ballonnet 10 lors de son gonflage. Il peut concrètement être constitué d'une pièce rapportée à l'extrémité distale de la semelle 30, constituée de tout matériau assurant la fonction de rigidité voulue. Ce matériau peut être choisi de façon non limitative parmi des polymères tels que du polychlorure de vinyle, du polysiloxane, du polyuréthane, du polycarbonate polyéthylène, du polyméthacrylate de méthyle, du polytéréphtalate d'éthylène, ou parmi des polymères fluorés tels que du polytétrafluoroéthylène ou du polychlorotrifluoroéthylène.

Le ballonnet 10 de cet autre mode de réalisation est représenté en conformation dégonflée sur la figure 5 et en conformation gonflée, avec retrait de la tige rigide 36 et du fil rigide amovible 38, sur la figure 6.

En référence à la figure 7, le dispositif de mesure 31 comporte tout d'abord un capteur externe 42 fixé sur une face externe 44 de la paroi 14 du corps de ballonnet 12 afin de venir au contact des tissus biologiques de la cavité nasale et/ou des fluides biologiques présents dans cette dernière lorsque le ballonnet 12 est gonflé dans la cavité. Le capteur externe 42 est conçu pour fournir, spontanément ou en réponse à une sollicitation électrique, un signal électrique de mesure sensible de la grandeur biologique mesurée. Le signal électrique de mesure est par exemple un signal analogique, tel qu'un courant électrique ou bien une tension électrique.

Par exemple, dans le cas où la grandeur biologique mesurée est le taux de glucose, le capteur externe 42 peut présenter deux électrodes. Ainsi, lorsqu'une sollicitation électrique sous la forme d'une tension électrique est appliquée au capteur externe 42, ce dernier fournit un signal électrique sous la forme d'un courant électrique dépendant du taux de glucose dans les tissus biologiques et/ou dans les fluides biologiques présents entre les électrodes.

Dans un autre exemple où le dispositif de mesure 31 fonctionne selon le principe de spectroscopie par réflectance diffuse (en anglais : « diffuse reflectance spectroscopy »), le capteur externe 42 peut comporter un émetteur d'un rayonnement électromagnétique (par exemple, de la lumière) destiné à interagir avec une masse tissulaire et au moins un récepteur du rayonnement électromagnétique ayant interagi avec la masse tissulaire. Le récepteur peut comprendre, sans s'y limiter, une ou plusieurs photodiodes. Un exemple de dispositif de spectroscopie par réflectance diffuse est par exemple décrit dans la demande de brevet américain publiée sous le numéro US 2011/0105865 A1.

Le capteur externe 42 présente de préférence une épaisseur de moins de 0,5 mm, de préférence encore de moins de 0,3 mm, afin d'affleurer le plus possible la paroi 14 du corps de ballonnet 12. Par exemple, le capteur externe 42 peut être un capteur imprimé sur la face externe 44 de la paroi 14 du corps de ballonnet 12. Dans ce cas, il présente généralement une épaisseur comprise entre 0,1 et 0,3 mm. Ce type de capteur présente en outre l'avantage de bien résister à la déformation du corps de ballonnet 12.

De préférence, la paroi 14 du corps de ballonnet 12 présente localement, au niveau du capteur externe 42, une portion 47, dite renfort, de plus forte rigidité que le reste de la paroi 14. En pratique, comme pour la semelle 30, le renfort 47 peut être une pièce rapportée contre la paroi 14 du corps de ballonnet 12, constituée de tout matériau assurant la fonction de rigidité voulue. De façon alternative et comme cela est représenté sur la figure 7, le renfort 47 peut être venu de matière avec le reste de la paroi 14 du corps de ballonnet 12, mais il présente alors une épaisseur sensiblement supérieure au reste de cette paroi 14 pour augmenter sa rigidité. Le renfort 47 permet d'éviter localement une trop forte déformation du corps de ballonnet 12 et donc une détérioration du capteur externe 42 lors du gonflage du ballonnet 10.

Le dispositif de mesure 31 comporte en outre un module interne 48 situé dans le corps de ballonnet 12 afin d'être protégé des fluides biologiques présents dans la cavité nasale. Le module interne 48 est par exemple fixé sur une face interne 50 de la paroi 14 du corps de ballonnet 12.

Le module interne 48 comporte un dispositif de traitement 52 destiné à recevoir et à traiter le signal électrique de mesure afin de fournir des mesures successives de la grandeur biologique, de préférence sous forme numérique. Le dispositif de traitement 52 comporte par exemple une carte de circuit imprimé. Lorsque le capteur externe 42 est conçu pour fournir le signal électrique de mesure en réponse à une sollicitation électrique, le dispositif de traitement 52 peut être conçu pour fournir cette sollicitation.

Le module interne 48 comporte en outre un dispositif de communication sans fil 54 conçu pour communiquer les mesures à un dispositif extérieur, de préférence un dispositif informatique, situé à l'extérieur du corps humain ou animal dans lequel le ballonnet 10 est inséré. Le dispositif informatique est par exemple un téléphone intelligent (de l'anglais « smartphone »), une tablette ou bien un ordinateur de bureau.

À la place ou en complément du dispositif de communication sans fil 54, le module interne 48 peut en outre comporter une mémoire 56 pour stocker les mesures, par exemple afin de les lire une fois le ballonnet 10 extrait de la cavité.

Le module électronique 48 peut en outre comporter une source d'énergie électrique 58, telle qu'une batterie chimique, pour alimenter ses éléments et en particulier le dispositif de traitement 52, le dispositif de communication sans fil 54 et/ou la mémoire 56.

Le capteur externe 42 transmet le signal électrique de mesure au module interne 48 au moyen d'un ou plusieurs fils électriques 60 passant dans un ou des conduits 62 ménagés au travers de la paroi 14 du corps de ballonnet 12.

L'étanchéité entre l'intérieur et l'extérieur du ballonnet 12 est assurée au moyen d'un matériau d'étanchéité 64, tel qu'un gel réticulé, obstruant le ou les conduits 62. Le matériau d'étanchéité 64 comporte par exemple de la colle permettant, outre sa fonction d'étanchéité, la fixation du capteur 42 à la face externe 44 de la paroi et/ou la fixation du module interne 48 à la face interne 50 de la paroi 14.

Par ailleurs, un ou plusieurs marqueurs radio-opaques 66 peuvent être prévus sur la paroi 14 du corps de ballonnet 12, par exemple sur sa face externe 44. Un marqueur radio-opaque 66 est par exemple prévu à proximité de chaque capteur externe 42, par exemple à moins d'un millimètre. Ainsi, il est possible de visualiser et de contrôler la position du corps de ballonnet 12 et/ou du ou des capteurs externes 42 afin de s'assurer qu'ils sont bien placés pour remplir leur fonction correctement. Lorsque les capteurs externes 42 sont directement visibles par imagerie (IRM, scanner, radioscopie, échographie, etc.), il est possible de se passer des marqueurs radio-opaques 66.

La présence du ou des dispositifs de mesure 31 sur le corps de ballonnet 12 permet ainsi la surveillance à distance de différents paramètres biologiques, dont la connaissance est utile en otorhinolaryngologie, neurochirurgie ou toute autre spécialité médicale.

Un logiciel dédié peut être programmé pour détecter une déviation des mesures par rapport à un intervalle normal dans lequel elles devraient se trouver. En cas de déviation détectée, une alerte automatique peut être envoyée (par exemple par courriel ou par message téléphonique court (SMS)) au professionnel de santé pour qu'il prévienne par exemple le patient chez qui l'implant 10 est posé. Alternativement ou bien en complément, l'alerte automatique peut être directement envoyée au patient, ou bien encore à un centre de traitement d'appels qui se chargera d'appeler le professionnel de santé et/ou le patient. Une fois les mesures obtenues, elles peuvent être utilisées par le professionnel de santé en vue de réagir à un évènement potentiellement non prévu une fois le patient sorti de l'hôpital, ou de poser ou d'affiner son diagnostic permettant ainsi de personnaliser le traitement du patient. Par ailleurs, les mesures obtenues peuvent être utilisées pour constituer ou bien alimenter des registres ou tout autre type de base de données.

Il apparaît clairement qu'un ballonnet gonflable à usage médical tel que celui décrit précédemment permet l'obtention de mesures de grandeurs biologiques, sans que leur présence ne vienne perturber l'écoulement des fluides biologiques ou abîmer les tissus biologiques.

Compte tenu de sa structure simple, il est en outre de conception et fabrication aisées, de sorte qu'il peut être à utilisation unique puis jeté.

Il est particulièrement adapté à des opérations médicales de chirurgies otorhinolaryngologiques, cervico-faciales ou bien en neurochirurgie. Il peut aussi s'utiliser dans d'autres opérations chirurgicales, notamment en orthopédie ou chirurgie digestive. Sans même parler de chirurgie, il peut aussi s'utiliser en tant que simple dispositif de compression pour traiter toutes sortes d'épistaxis ou saignements de fosses nasales, de tels saignements se produisant par exemple couramment en consultation.

On notera par ailleurs que l'invention n'est pas limitée aux modes de réalisation décrits précédemment. L'invention est définie dans les revendications.

En particulier, les formes de la semelle 30 et du renfort distal 40 peuvent être adaptées à toutes les applications souhaitées. Elles sont donc potentiellement très diverses.

## Revendications

1. Ballonnet gonflable (10) à usage médical, destiné à être inséré dégonflé dans une cavité de corps humain ou animal puis gonflé à l'intérieur de cette cavité en vue de faire pression contre elle, comportant :
- un corps de ballonnet (12) dont la paroi (14) est en matériau souple gonflable,
- une ouverture de gonflage (22) pour l'introduction d'un fluide dans le corps de ballonnet (12) de manière à le gonfler sous pression, et
- au moins un dispositif de mesure (31) d'une grandeur biologique de tissus biologiques de la cavité et/ou de fluides biologiques présents dans cette cavité, le dispositif de mesure (31) comportant :
- un capteur externe (42) fixé sur une face externe (44) de la paroi (14) du corps de ballonnet (12) et conçu pour fournir un signal électrique de mesure sensible à la grandeur biologique mesurée, et **caractérisé en ce que** le dispositif de mesure (31) comporte en outre :
- un module interne (48) situé à l'intérieur du corps de ballonnet (12) et comportant un dispositif de traitement (52) conçu pour traiter le signal électrique de mesure afin de fournir au moins une mesure de la grandeur biologique.

2. Ballonnet gonflable (10) à usage médical selon la revendication 1, dans lequel le capteur externe (42) transmet le signal électrique de mesure au module interne (48) au moyen d'un ou plusieurs fils électriques (60) passant dans un ou des conduits (62) ménagés au travers de la paroi (14) du corps de ballonnet (12).

3. Ballonnet gonflable (10) à usage médical selon la revendication 2, dans lequel un matériau d'étanchéité (64) obstrue le ou les conduits (62).

4. Ballonnet gonflable (10) à usage médical selon la revendication 3, dans lequel le matériau d'étanchéité (62) comporte de la colle fixant le capteur (42) à la face externe (44) de la paroi (14) du corps de ballonnet (12).

5. Ballonnet gonflable (10) à usage médical selon l'une quelconque des revendications 1 à 4, dans lequel le module interne (48) est fixé sur une face interne (50) de la paroi (14) du corps de ballonnet (12).

6. Ballonnet gonflable (10) à usage médical selon les revendications 3 et 5 prises ensemble, dans lequel le matériau d'étanchéité (62) comporte de la colle fixant le module interne (48) à la face interne (50) de la paroi (14) du corps de ballonnet (12).

7. Ballonnet gonflable (10) à usage médical selon l'une quelconque des revendications 1 à 6, dans lequel le module interne (48) comporte en outre un dispositif de communication sans fil (54) conçu pour communiquer la mesure de la grandeur biologique à un dispositif situé à l'extérieur du corps humain ou animal dans lequel le ballonnet gonflable (10) est inséré.

8. Ballonnet gonflable (10) à usage médical selon l'une quelconque des revendications 1 à 7, dans lequel le module interne (48) comporte en outre une mémoire (56) pour stocker la mesure de la grandeur biologique.

9. Ballonnet gonflable (10) à usage médical selon l'une quelconque des revendications 1 à 8, dans lequel le capteur externe (42) est imprimé sur la face externe (44) de la paroi (14) du corps de ballonnet (12).

10. Ballonnet gonflable (10) à usage médical selon l'une quelconque des revendications 1 à 9, à utilisation en otorhinolaryngologie ou en neurochirurgie, dans lequel le corps de ballonnet (12) est préformé pour épouser les formes intérieures d'une cavité nasale (32) de corps humain ou animal lorsqu'il est gonflé.

## Patentansprüche

1. Aufblasbarer Ballon (10) für medizinische Zwecke, der dazu bestimmt ist, unaufgeblasen in eine menschliche oder tierische Körperhöhle eingeführt und dann im Inneren dieser Höhle aufgeblasen zu werden, um Druck auf sie auszuüben, umfassend:
- einen Ballonkörper (12), dessen Wand (14) aus weichem, aufblasbarem Material besteht,
- eine Aufblasöffnung (22) zum Einleiten eines Fluids in den Ballonkörper (12), um ihn unter Druck aufzublasen, und
- mindestens eine Vorrichtung zum Messen (31) einer biologischen Größe von biologischen Geweben der Höhle und/oder von biologischen Fluiden, die in dieser Höhle vorhanden sind,
wobei die Messvorrichtung (31) umfasst:
- einen externen Sensor (42), der an einer Außenseite (44) der Wand (14) des Ballonkörpers (12) befestigt und dafür ausgelegt ist, ein elektrisches Messsignal zu liefern, das auf die gemessene biologische Größe anspricht, und
**dadurch gekennzeichnet, dass** die Messvorrichtung (31) weiter umfasst:
- ein internes Modul (48), das sich im Inneren des Ballonkörpers (12) befindet und eine Verarbeitungsvorrichtung (52) umfasst, die dafür ausgelegt ist, das elektrische Messsignal zu verarbeiten, um mindestens eine Messung der biologischen Größe zu liefern.

2. Aufblasbarer Ballon (10) für medizinische Zwecke nach Anspruch 1, wobei der externe Sensor (42) das elektrische Messsignal mittels eines oder mehrerer elektrischer Drähte (60), die in einem oder mehreren durch die Wand (14) des Ballonkörpers (12) ausgebildeten Kanälen (62) verlaufen, an das interne Modul (48) überträgt.

3. Aufblasbarer Ballon (10) für medizinische Zwecke nach Anspruch 2, wobei ein Dichtungsmaterial (64) den oder die Kanäle (62) verschließt.

4. Aufblasbarer Ballon (10) für medizinische Zwecke nach Anspruch 3, wobei das Dichtungsmaterial (62) Klebstoff umfasst, der den Sensor (42) an der Außenseite (44) der Wand (14) des Ballonkörpers (12) befestigt.

5. Aufblasbarer Ballon (10) für medizinische Zwecke nach einem der Ansprüche 1 bis 4, wobei das interne Modul (48) an einer Innenseite (50) der Wand (14) des Ballonkörpers (12) befestigt ist.

6. Aufblasbarer Ballon (10) für medizinische Zwecke nach den Ansprüchen 3 und 5 in Kombination, wobei das Dichtungsmaterial (62) Klebstoff umfasst, der das interne Modul (48) an der Innenseite (50) der Wand (14) des Ballonkörpers (12) befestigt.

7. Aufblasbarer Ballon (10) für medizinische Zwecke nach einem der Ansprüche 1 bis 6, wobei das interne Modul (48) weiter eine Drahtloskommunikationsvorrichtung (54) umfasst, die dafür ausgelegt ist, die Messung der biologischen Größe an eine Vorrichtung zu kommunizieren, die sich außerhalb des menschlichen oder tierischen Körpers, in den der aufblasbare Ballon (10) eingeführt wird, befindet.

8. Aufblasbarer Ballon (10) für medizinische Zwecke nach einem der Ansprüche 1 bis 7, wobei das interne Modul (48) weiter einen Speicher (56) zum Speichern der Messung der biologischen Größe umfasst.

9. Aufblasbarer Ballon (10) für medizinische Zwecke nach einem der Ansprüche 1 bis 8, wobei der externe Sensor (42) auf die Außenseite (44) der Wand (14) des Ballonkörpers (12) gedruckt ist.

10. Aufblasbarer Ballon (10) für medizinische Zwecke nach einem der Ansprüche 1 bis 9 zur Verwendung in der Hals-Nasen-Ohren-Heilkunde oder in der Neurochirurgie, wobei der Ballonkörper (12) vorgeformt ist, um sich, wenn er aufgeblasen wird, an die inneren Formen einer Nasenhöhle (32) eines menschlichen oder tierischen Körpers anzuformen.

## Claims

1. An inflatable balloon (10) for medical use, intended to be inserted deflated into a cavity of a human or animal body then inflated inside this cavity in order to press against it, including:
- a balloon body (12) the wall (14) of which is made of flexible inflatable material,
- an inflation opening (22) for the introduction of a fluid into the balloon body (12) so as to inflate it under pressure, and
- at least one device (31) for measuring a biological quantity of biological tissues of the cavity and/or of biological fluids present in this cavity,
the measuring device (31) including:
- an external sensor (42) attached on an external face (44) of the wall (14) of the balloon body (12) and designed to supply an electrical measurement signal sensitive to the measured biological quantity, and
**characterized in that** the measuring device (31) further includes:
- an internal module (48) located inside the balloon body (12) and including a processing device (52) designed to process the electrical measurement signal in order to provide at least one measurement of the biological quantity.

2. The inflatable balloon (10) for medical use according to claim 1, wherein the external sensor (42) transmits the electrical measurement signal to the internal module (48) by means of one or more electrical wire(s) (60) passing through conduit(s) (62) formed through the wall (14) of the balloon body (12).

3. The inflatable balloon (10) for medical use according to claim 2, wherein a sealing material (64) obstructs the conduit(s) (62).

4. The inflatable balloon (10) for medical use according to claim 3, wherein the sealing material (62) includes a glue attaching the sensor (42) to the external face (44) of the wall (14) of the balloon body (12).

5. The inflatable balloon (10) for medical use according to any one of claims 1 to 4, wherein the internal module (48) is attached on an internal face (50) of the wall (14) of the balloon body (12).

6. The inflatable balloon (10) for medical use according to claims 3 and 5 taken together, wherein the sealing material (62) includes a glue attaching the internal module (48) to the internal face (50) of the wall (14) of the balloon body (12).

7. The inflatable balloon (10) for medical use according to any one of claims 1 to 6, wherein the internal module (48) further includes a wireless communication device (54) designed to communicate the measurement of the biological quantity to a device located outside the human or animal body whereinto the inflatable balloon (10) is inserted.

8. The inflatable balloon (10) for medical use according to any one of claims 1 to 7, wherein the internal module (48) further includes a memory (56) for storing the biological quantity measurement.

9. The inflatable balloon (10) for medical use according to any one of claims 1 to 8, wherein the external sensor (42) is printed on the external face (44) of the wall (14) of the balloon body (12).

10. The inflatable balloon (10) for medical use according to any one of claims 1 to 9, for a use in otorhinolaryngology or neurosurgery, wherein the balloon body (12) is pre-formed to conform to the internal shapes of a nasal cavity (32) of a human or animal body when it is inflated.
